(19) Europäisches Patentamt
European Patent Office
Office européen des brevets

(11) Numéro de publication : **0 150 137**
**B1**

(12) # FASCICULE DE BREVET EUROPÉEN

(45) Date de publication du fascicule du brevet : **21.10.87**

(51) Int. Cl.[4] : **C 01 G 55/00**, B 01 J 31/24

(21) Numéro de dépôt : **85400012.2**

(22) Date de dépôt : **07.01.85**

(54) **Clusters mixtes à base de palladium et fer utilisables comme catalyseurs, leur procédé de fabrication.**

(30) Priorité : **17.01.84 FR 8400630**

(43) Date de publication de la demande :
**31.07.85 Bulletin 85/31**

(45) Mention de la délivrance du brevet :
**21.10.87 Bulletin 87/43**

(84) Etats contractants désignés :
**BE CH DE FR GB IT LI LU NL SE**

(56) Documents cités :
**EP-A- 0 031 877**
**EP-A- 0 078 729**
**J.J. BURTON et al.: "ADVANCED MATERIALS IN CATALYSIS", 1977, pages 33-65, Academic Press, New York, US; Chapitre 2: "Physical and chemical properties of supported bimetallic catalysts"**

(73) Titulaire : **ATOCHEM**
**4 & 8, Cours Michelet La Défense 10**
**F-92800 Puteaux (FR)**

(72) Inventeur : **Braunstein, Pierre**
**13, Rue du Faubourg de Saverne**
**F-67000 Strasbourg (FR)**
Inventeur : **Kervennal, Jacques**
**134, Rue E. Locard**
**F-69005 Lyon (FR)**
Inventeur : **Richert, Jean-Luc**
**10, Rue Kellermann**
**F-67300 Schiltigheim (FR)**
Inventeur : **Ries, Michel**
**38, Boulevard Clémenceau**
**F-67000 Strasbourg (FR)**

(74) Mandataire : **Foiret, Claude et al**
**ATOCHEM Département Propriété Industrielle**
**F-92091 Paris la Défense 10 Cedex 42 (FR)**

**EP 0 150 137 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux clusters hétéropolymétalliques contenant du palladium et du fer, elle concerne également leur synthèse. On entend par clusters, des édifices moléculaires comportant au moins trois atomes métalliques reliés les uns aux autres par des liaisons métal-métal, ces édifices possèdant des ligands qui, par leur structure, les stabilisent plus ou moins. De telles espèces clusters sont particulièrement intéressantes pour élaborer des catalyseurs en les imprégnant sur un support, puis en traitant thermiquement et en réduisant pour obtenir des particules métalliques. Dans le EP-A 0 078 729 est décrite l'utilisation, sous forme de dépôts sur un support, de clusters mixtes bi-métalliques, à base par exemple de palladium et fer, en tant que précurseurs de la phase active de catalyseurs pour la carbonylation de composés nitroaromatiques. On peut aussi considérer comme clusters conformes à l'invention les complexes mixtes de formule générale $Pd_2(Fe)_x(CO)_4\ [CO(NO)_2]_y\ (dppm)_2$ avec x = 2 quand y = 1 et x = 1 quand y = 0, dppm désignant le ligand bis (diphénylphosphino) méthane. L'association du fer et du palladium permet d'obtenir des résultats intéressants dans certaines réactions catalytiques ; il est alors important que la distribution et la dispersion des métaux soient les meilleures possibles. Dans le cas de catalyseurs bimétalliques contenant du fer supporté, la structure du catalyseur et l'état de valence des métaux sont évidemment primordiaux. C. H. Bartholomew et M. Boudart ont montré dans le Journal of Catalysis, 29, 278 (1973) que les étapes d'imprégnation, de cuisson et de réduction, de même que la nature du support, sont très importantes pour obtenir des catalyseurs très dispersés. En effet, si l'interaction métal-support est faible, les particules métalliques produites peuvent migrer aisément à la surface et former de gros cristallites ; ainsi sur silice, si on prépare un catalyseur par imprégnation d'une solution aqueuse de nitrate de fer, l'interaction métal-support est faible et conduit à de gros cristallites, de taille moyenne 15 $\mu m$, comme l'ont montré L. Guczi et coll. dans le Journal of Catalysis 60, 121 (1979) et R. L. Garten dans Mössbauer Eff. Method, 10, 69 (1977). Le fait de partir de sels métalliques en solutions aqueuses rend importants les phénomènes de pH qui affectent l'interaction entre la surface et l'ion et peuvent conduire à des précipitations en surface. L'interaction métal-support est beaucoup plus importante dans le cas de l'alumine comme le montrent R. L. Garten et D. F. Ollis dans le Journal of Catalysis, 35, 232 (1974) et on peut préparer un catalyseur très dispersé, mais par contre, en partant d'une espèce de Fe(III), la réduction en Fe(0) en peut être effectuée totalement. Une possibilité de produire un catalyseur où le fer est très dispersé et dans un état d'oxydation proche de 0 est donc de déposer du fer de valence faible sur le support ; ceci peut être réalisé en utilisant des métaux carbonyles comme l'ont montré A. Brenner dans le Journal of Molecular Catalysis, 5, 157 (1979) et J. M. Basset et R. Ugo dans Aspects of Homogeneous Catalysis, vol. II, 137, Reidel (1976). Dans le cas où on recherche des catalyseurs bi-métalliques, on voit l'avantage que l'on a de partir d'espèces hétéropolynucléaires, telles que les clusters mixtes, dans lesquelles les ligands confèrent aux métaux de faibles degrés d'oxydation. En effet, les catalyseurs bi-métalliques conventionnels sont préparés par imprégnation d'une solution aqueuse des sels. Les imprégnations de sels peuvent d'ailleurs être simultanées ou successives, mais là encore des modifications de pH au cours de l'imprégnation peuvent provoquer des processus d'agrégation métallique et donc une mauvaise dispersion des phases. De plus, les vitesses d'adsorption ne sont généralement pas identiques et dans le cas où on imprègne sur des supports préformés il apparaît des gradients de concentration qui sont différents pour les deux métaux.

Ces inconvénients disparaissent avec des clusters moléculaires bi-métalliques du fait que probablement ils conduisent à des phases hétérométalliques bien dispersées et uniformes, les clusters imprégnés pouvant garder les géométries des complexes de départ. La formation d'agrégats bi-métalliques de surface, à partir d'imprégnations classiques de dérivés de platine ou palladium et de fer, a toutefois été mise en évidence et décrite par J. J. Burton et R. LM. Garten dans Advanced Materials in Catalysis, Academic, New York, 33 (1977). Mais dans ce cas, on n'est pas maître de la stœchiométrie des espèces formées ; au contraire, le fait de partir de complexes moléculaires, dans lesquels les liaisons métal-métal sont stables, permet de mieux contrôler les arrangements formés et leur stœchiométrie.

Très peu d'édifices moléculaires contenant des liaisons palladium-fer ont été caractérisés à ce jour. On peut citer le complexe $[Fe\ Pd(\mu Cl)\ (\mu PPh)_2\ (CO)_4]_2$ préparé par B. C. Benson, R. Jackson, K. K. Joshi et D. T. Thompson d'après la description qu'ils ont faite dans Chemical Communications, 1506 (1968) ainsi que les espèces $[Fe_4Pd(CO)_{16}]^{2-}$ ; $[Fe_6Pd_6H(CO)_{24}]^{3-}$ et $[Fe_6Pd_6(CO)_{24}]^{4-}$ décrites par G. Longoni, M. Manassero et M. Sansoni dans le Journal of the American Chemical Society vol. 102, 3242 (1980). En fait, seuls les trois derniers complexes répondent à la définition de clusters donnée au début de ce descriptif et possèdent des liaisons palladium-palladium stabilisant l'édifice moléculaire, mais il convient de noter qu'il s'agit d'espèces ioniques, ce facteur pouvant gêner ou compliquer leur utilisation éventuelle comme catalyseurs ou précurseurs de catalyseurs puisqu'on ne peut éviter l'introduction simultanée du contre-ion correspondant, ce qui a pour conséquence de faire intervenir un paramètre supplémentaire dont on ne peut s'affranchir.

Les systèmes revendiqués présentent l'avantage, outre le fait d'être neutres, de posséder des ligands diphosphines qui par leur caractère bidenté, renforcent la solidité des structures. La synthèse des clusters de l'invention fait appel à plusieurs étapes réactionnelles ; ainsi pour le palladium, il faut d'abord préparer le complexe $PdCl_2(PhCN)_2$ par réaction de $PdCl_2$ et de benzonitrile comme décrite par F.A.

Hartley dans Organometal Chem. Rev., 6, 119, (1970) et le complexe $Pd_2(dba)_3$, $CHCl_3$(dba) : dibenzylidèneacétone) par réaction de $PdCl_2$ et de dibenzylidène acétone comme décrite par T. Ukai, H. Kawazura, Y. Ishii, J. Bonnet, J. A. Ibers dans J. Organomet. Chem. 65, 253 (1974). La mise en réaction de ces deux espèces en présence de bis (diphénylphosphino) méthane (dppm) conduit au complexe $Pd_2Cl_2(dppm)_2$ selon l'équation :

$$Pd_2(dba)_3, CHCl_3 + 2PdCl_2(PhCN)_2 + 4\ dppm \rightarrow 2Pd_2Cl_2(dppm)_2 + 3\ dba + CHCl_3 + 4\ PhCN$$

(voir L. S. Benner et A. L. Balch. J. Amer. Chem. Soc., 100, 6099 (1978).

Ce complexe est utilisé comme précurseur pour synthétiser les clusters de l'invention en le faisant réagir avec un composé $MFe(CO)_3X$, M étant un métal alcalin et X pouvant être CO ou NO. Ainsi $KFe(CO)_3NO$ que l'on peut préparer à partir de $Fe(CO)_5$ et $KNO_2$ comme décrit par W. Hieber et H. Beutner dans Z. Naturforsch. Col. 15B, 323 (1960) et $Na_2Fe(CO)_4$ décrit par J. P. Collman et coll. dans le J. Amer. Chem. Soc. 94, 1788 (1972) et dans Accounts Chem. Res. Vol. 8, 342 (1975) peuvent réagir avec $Pd_2Cl_2(dppm)_2$ pour conduire respectivement aux deux espèces $Pd_2Fe_2(dppm)_2$ $(CO)_5$ $(NO)_2$ et $Pd_2Fe(CO)_4$ $(dppm)_2$. On peut noter que ces deux complexes permettent de faire varier le rapport atomique Pd/Fe. La réaction s'effectue habituellement en présence de solvants inertes comme par exemple le tétrahydrofurane (THF) ou le dioxanne à des températures inférieures ou égales à 0 °C.

La structure des clusters a pu être déterminée sur des bases analytiques et spectroscopiques :

a) $Pd_2Fe_2$ $(dppm)_2$ $(CO)_5$ $(NO_2)$ : l'analyse élémentaire est conforme à cette formule générale et les données de résonance magnétique nucléaire (R. M. N.) confirment également un complexe tétramétallique mixte ayant deux atomes de palladium et deux atomes de fer et présentant une géométrie triangulaire.

RMN $^1H(CDCl_3)$ :

= 7,7 — 6,8 ppm : multiplet des 40 protons aromatiques de bis(diphénylphosphino) méthane
= 4,50 ppm : « doublet de triplets » correspondant à deux protons de $CH_2$
= 3,91 ppm : « triplet » correspondant à deux protons de $CH_2$

RMN $^{31}P(CDCl_3$ — référence : $H_3PO_4$ 85 %)

= + 40 ppm : multiplet correspondant à un atome de phosphore
= + 4,6 à — 9,1 ppm : ensemble de 22 raies, correspondant à 3 atomes de phosphore

En première analyse, la RMN du proton montre au niveau des groupements méthylène un triplet dû au $CH_2$ du ligand dppm pontant la liaison Pd-Pd et un doublet de triplet, à champ plus faible, dû au $CH_2$ pontant la liaison Pd-Fe, qui se couple à un P d'un pont voisin.

La RMN du $^{31}p$ confirme une structure triangulaire montrant 1 atome de phosphore lié à un atome de Fe et très différent des 3 autres P liés aux atomes de palladium. Ceci amène à attribuer au cluster la structure suivante :

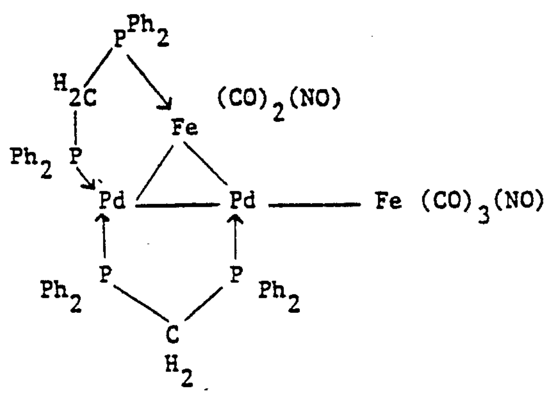

b) $Pd_2Fe(Co)_4$ $(dppm)_2$ : L'analyse élémentaire est conforme à cette formule générale les spectres de RMN suggèrent un complexe trimétallique mixte de géométrie triangulaire :

RMN $^1H$ $(CD_2Cl_2)$ :

$\delta$ = 7,5 — 6,4 ppm : multiplet des 40 protons aromatiques de bis-(diphénylphosphino) méthane
$\delta$ = 4,4 ppm : centre d'un massif d'aspect triplet correspondant à deux protons de $CH_2$
$\delta$ = 4,2 ppm : centre d'un deuxième massif d'aspect triplet correspondant à deux protons de $CH_2$

RMN $^{31}p$ $(CD_2Cl_2$ — référence : $H_3PO_4$ 85 %)

δ = + 54,3 ppm : centre d'un massif de 6 raies correspondant à 1 atome de phosphore
δ = — 2,9 à — 8,6 ppm : massif de 15 raies, correspondant à 3 atomes de phosphore.

Ces résultats permettent d'attribuer au cluster la structure suivante :

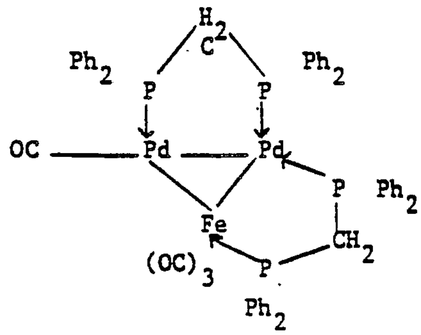

Les supports qui peuvent être utilisés pour préparer les catalyseurs sont préférentiellement les alumines, silice-alumines, silices, charbons, oxyde de molybdène, etc. Les catalyseurs peuvent être préparés par un procédé comprenant les étapes suivantes :

— imprégnation du support avec une solution du cluster dans un solvant organique
— séchage du support imprégné et cuisson
— réduction en fin de traitement thermique pour libérer les particules métalliques.

L'imprégnation des supports a été effectuée dans un évaporateur rotatif. La solution du cluster est soit ajoutée en une seule fois sur le support et le solvant chassé sous pression réduite, soit introduite lentement sur le support de façon à déposer sur le support une quantité de palladium comprise entre 0,1 et 10 % en poids, le fer étant présent à un pourcentage en poids correspondant à la stœchiométrie du cluster. Le traitement thermique peut être effectué par chauffage sous courant d'azote de 20 °C jusqu'à une température comprise entre 450 et 500 °C au moyen d'une programmation de température, suivi d'un palier de plusieurs heures, environ 10 à 30, à la température finale, un balayage d'hydrogène étant admis à la fin du palier. Nous donnons ci-dessous à titre non limitatif un exemple d'application d'un tel catalyseur à la carbonylation de l'ortho nitrophénol en benzoxazolone-2.

Par précaution, les manipulations de complexe sont effectuées sous azote ou argon et les solvants séchés et distillés.

## Exemple 1

Une solution de $KFe(CO)_3NO$ (2,8 g, 13,4 mmoles), préparée selon la description faite par W. Hieber et H. Beutner dans Z. Naturforsch. Vol. 15 B, 323 (1960), dans 500 ml de THF est ajoutée goutte à goutte à une suspension de $Pd_2(dppm)_2Cl_2$ (6,46 g ; 6,1 mmoles), dont la synthèse a été décrite par L. S. Benner et A. L. Balch dans J. Amer. Chem. Soc., 100, 6099 (1978) et placée dans 500 ml de THF à — 78 °C. Le mélange est protégé de la lumière. On laisse remonter la température jusqu'à 0 °C : le mélange passe progressivement de l'orange au rouge foncé, puis au violet au bout de 3 heures. On laisse alors revenir à température ambiante tout en maintenant l'agitation jusqu'à coloration verte.

Le mélange réactionnel est filtré sur fritté afin d'éliminer KCl, des produits de décomposition et $Pd_2(dppm)_2$ ($\mu$CO) $Cl_2$ qui s'est formé. Le filtrat vert foncé est concentré et mis à précipiter avec de l'hexane. On récupère ainsi 5,04 g de solide vert foncé (rendement 64 % sur Pd), recristallisable dans le mélange $CH_2Cl_2$/hexane.

Analyse élémentaire :
théorique : C = 51,07 % ; H = 3,43 % ; N = 2,16 %
trouvé : C = 50,6 % ; H = 3,75 % ; N = 1,91 %
bandes en infra-rouge (pastille KBr) :
1 974 $cm^{-1}$ (intense)
1 880 $cm^{-1}$ (intense, large)
1 840 $cm^{-1}$ (épaulement)
1 736 $cm^{-1}$ (intense)
1 683 $cm^{-1}$ (intense)
Point de fusion : 178 °C

Les données d'analyse et de RMN conduisent à attribuer au cluster formé la formule $Pd_2Fe_2(dppm)_2(CO)_5(NO)_2$.

## Exemple 2

On introduit dans un ballon sous argon 1,2 g de $Na_2Fe(CO)_4$, 3/2 dioxanne soit 3,48 mmoles et 3,66 g

de $Pd_2Cl_2(dppm)_2$ (3,48 mmoles) puis on refroidit à —78 °C et on ajoute 325 ml de THF préalablement refroidi à —78 °C. La suspension est alors agitée. On laisse la température remonter progressivement jusqu'à 0 °C. La réaction est suivie par infra-rouge en observant l'évolution des bandes du produit dans la zone des CO. On concentre alors la solution jusqu'à 10 ml environ. On ajoute 430 ml de pentane refroidi à —78 °C en continuant à agiter. Le mélange est placé 1 heure dans la carboglace. Après filtration, le solide est lavé avec du pentane, puis avec de l'eau distillée et séché. Il est recristallisé du dichlorométhane-pentane pour donner 3 g de cristaux bruns (rendement 75 % en Pd).

Analyse élémentaire :
théorique : C = 56,4 % ; H = 3,86 %
trouvé : C = 55,4 % ; H = 3,93 %
bandes en infra-rouge (solution dans THF) :
2 024 $cm^{-1}$ (intense)
1 964 $cm^{-1}$ (faible)
1 939 $cm^{-1}$ (faible)
1 916 $cm^{-1}$ (intense)
1 868 $cm^{-1}$ (faible)
1 826 $cm^{-1}$ (intense)
Point de fusion : 160 °C (décomposition)

Les données d'analyse et de RMN conduisent à attribuer au cluster formé la formule $Pd_2Fe(CO)_4$ $(dppm)_2$.

## Exemple 3

On prépare un catalyseur à partir du cluster $Pd_2Fe(CO)_4$ $(dppm)_2$ dont la synthèse a été décrite dans l'exemple 2. Pour cela, on place dans un ballon à solides monté sur un évaporateur rotatif, 9 g de billes de diamètre 2,6 mm, d'un support silice de surface spécifique supérieure à 50 $m^2/g$ et de volume poreux supérieur à 0,7 $cm^3/g$ préalablement traité pendant une heure à 300 °C sous azote. On dégaze le support, puis introduit lentement à l'aide d'un capillaire une solution de 1 g du cluster dans 100 ml de THF fraîchement distillé sur sodium et désoxygéné. On opère sous pression réduite de 250 mm Hg en aspergeant le support, à une température telle que le solvant soit évaporé au fur et à mesure de l'imprégnation. Après imprégnation, on sèche le support imprégné puis le place dans un tube de cuisson pour le traiter thermiquement. On fait passer un courant d'azote puis élève la température jusqu'à 450 °C à raison de 40 °C/h. On laisse 15 heures en palier, puis introduit un courant d'hydrogène pendant une heure. Après refroidissement, le catalyseur est analysé : la teneur en palladium est de 1,9 % ; celle en fer de 0,5 %.

## Exemple 4

Dans un autoclave de 500 ml muni d'une agitation magnétique, on introduit 7 g d'orthonitrophénol (0,05 mole) qu'on dissout dans 100 ml d'orthodichlorobenzène et 1 g de pyridine. On ajoute 2,75 g du catalyseur synthétisé dans l'exemple 3 (on utilise donc un rapport molaire $NO_2/Pd$ de 100). Après balayage du réacteur à l'azote, on introduit 200 bars de monoxyde de carbone puis chauffe à 200 °C pendant 3 H 30. Après refroidissement, on filtre le catalyseur qu'on récupère intégralement et dose le filtrat : le taux de transformation globale de l'orthonitrophénol est de 98,4 % et la sélectivité en benzoxazolone-2 formée de 92 %.

**Revendications**

1. Cluster hétéropolymétallique de formule $Pd_2(Fe)_x(CO)_4$ $[CO(NO)_2]_y$ $(dppm)_2$ avec x = 2 quand y = 1 et x = 1 quand y = 0, dppm désignant le ligand bis(diphénylphosphino) méthane.

2. Cluster hétérotétramétallique selon la revendication 1 de formule $Pd_2Fe_2(dppm)_2(CO)_5(NO)_2$ de point de fusion de 178 °C et présentant les bandes caractéristiques suivant en spectroscopie infra-rouge (pastille KBr) :

1 974 $cm^{-1}$ intense
1 880 $cm^{-1}$ intense
1 840 $cm^{-1}$ épaulement
1 736 $cm^{-1}$ intense
1 683 $cm^{-1}$ intense

3. Cluster hétérotrimétallique selon la revendication 1 de formule $Pd_2Fe(CO)_4$ $(dppm)_2$ de point de fusion de 160 °C et présentant les bandes caractéristiques suivantes en spectroscopie infrarouge (en solution dans le tétrahydrofurane) :

2 024 $cm^{-1}$ intense
1 964 $cm^{-1}$ faible
1 939 $cm^{-1}$ faible
1 916 $cm^{-1}$ intense
1 868 $cm^{-1}$ faible
1 826 $cm^{-1}$ intense

4. Procédé de fabrication des clusters des revendications 1 à 3 caractérisé en ce qu'on fait réagir le complexe $Pd_2Cl_2(dppm)_2$ sur un composé $MFe(CO)_3X$ dans lequel M est un métal alcalin, X pouvant être CO ou NO.

5. Procédé de fabrication selon la revendication 4 caractérisé en ce que $MFe(CO)_3X$ est $KFe(CO)_3NO$ ou $Na_2Fe(CO)_4$.

6. Procédé selon l'une des revendications 4 à 5 caractérisé en ce que la réaction s'effectue en présence de solvant inerte.

7. Procédé selon l'une des revendications 4 à 6 caractérisé en ce que la température de réaction est inférieure ou égale à 0 °C.

8. Application des clusters des revendications 1 à 3 à la fabrication de catalyseur par imprégnation desdits clusters sur un support suivie d'un traitement thermique et d'une réduction.

**Claims**

1. Heteropolymetallic cluster of formula $Pd_2(Fe)_x\ (CO)_4\ [CO(NO)_2]_y\ (dppm)_2$ with $x = 2$ when $y = 1$ and $x = 1$ when $y = 0$, dppm denoting the bis(diphenylphosphino)-methane ligand.

2. Heterotetrametallic cluster according to claim 1, of formula $Pd_2Fe_2(dppm)_2\ (CO)_5\ (NO)_2$, with a melting point of 178 °C and having the following characteristic bands in infrared spectroscopy (KBr disc) :

1 974 $cm^{-1}$ strong
1 880 $cm^{-1}$ strong
1 840 $cm^{-1}$ shoulder
1 736 $cm^{-1}$ strong
1 683 $cm^{-1}$ strong

3. Heterotrimetallic cluster according to claim 1, of formula $Pd_2Fe(Co)_4\ (dppm)_2$, with a melting point of 160 °C and having the following characteristic bands in infrared spectroscopy (dissolved in tetrahydrofuran) :

2 024 $cm^{-1}$ strong
1 964 $cm^{-1}$ weak
1 939 $cm^{-1}$ weak
1 916 $cm^{-1}$ strong
1 868 $cm^{-1}$ weak
1 826 $cm^{-1}$ strong

4. Process for the manufacture of the clusters in claims 1 to 3, characterized in that the complex $Pd_2Cl_2(dppm)_2$ is reacted with a compound $MFe(CO)_3X$ in which M is an alkali metal, it being possible for X to be CO or NO.

5. Manufacturing process according to claim 4, characterized in that $MFe(CO)_3X$ is $KFe(CO)_3NO$ or $Na_2Fe(CO)_4$.

6. Process according to one of claims 4 to 5, characterized in that the reaction is carried out in the presence of an inert solvent.

7. Process according to one of claims 4 to 6, characterized in that the reaction temperature is less than or equal to 0 °C.

8. Application of the clusters in claims 1 to 3 to the manufacture of a catalyst by impregnating the said clusters onto a support followed by a heat treatment and a reduction.

**Patentansprüche**

1. Heteropolymetall-Cluster der Formel $Pd_2(Fe)_x(CO)_4\ [CO(NO)_2]_y\ (dppm)_2$ mit $x = 2$, wenn $y = 1$ und $x = 1$, wenn $y = 0$, wobei dppm für den Liganden bis(Diphenylphosphino) methan steht.

2. Heterotetrametall-Cluster nach Anspruch 1 mit der Formel $Pd_2Fe_2(dppm)_2\ (CO_5)\ (NO)_2$, mit Schmelzpunkt 178 °C sowie den nachfolgenden charakteristischen Banden im Infrarotspektrum (KBr-Tablette) :

1 974 $cm^{-1}$ intensiv
1 880 $cm^{-1}$ intensiv
1 840 $cm^{-1}$ Schulter
1 736 $cm^{-1}$ intensiv
1 683 $cm^{-1}$ intensiv

3. Heterotrimetall-Cluster nach Anspruch 1 mit der Formel $Pd_2Fe(CO)_4$ $(dppm)_2$, dem Schmelzpunkt 160 °C sowie den nachfolgenden charakteristischen Banden im Infrarotspektrum (in Tetrahydrofuranlösung) :

2 024 $cm^{-1}$ intensiv
1 964 $cm^{-1}$ schwach
1 939 $cm^{-1}$ schwach
1 916 $cm^{-1}$ intensiv
1 868 $cm^{-1}$ schwach
1 826 $cm^{-1}$ intensiv

4. Verfahren zur Herstellung von Clustern gemäß den Ansprüchen 1 bis 3, dadurch gekennzeichnet, daß man den Komplex $Pd_2Cl_2(dppm)_2$ mit einer Verbindung $MFe(CO)_3X$, in der M ein Alkalimetall ist und X CO oder NO sein kann, reagieren läßt.

5. Herstellungsverfahren nach Anspruch 4, dadurch gekennzeichnet, daß $MFe(CO)_3X$ $KFe(CO)_3NO$ oder $Na_2Fe(CO)_4$ ist.

6. Verfahren gemäß einem der Ansprüche 4 oder 5, dadurch gekennzeichnet, daß man die Reaktion in Gegenwart eines inerten Solvens ausführt.

7. Verfahren gemäß einem der Ansprüche 4 bis 6, dadurch gekennzeichnet, daß die Reaktionstemperatur kleiner oder gleich 0 °C ist.

8. Verwendung von Clustern gemäß den Ansprüchen 1 bis 3 zur Herstellung eines Katalysators durch Aufbringen dieser Cluster auf einen Träger sowie nachfolgende thermische Behandlung und Reduktion.